# EUROPÄISCHE PATENTANMELDUNG

(11) **EP 2 857 013 A1**
(43) Veröffentlichungstag der Anmeldung: **08.04.2015**
(21) Anmeldenummer: 14196353.8
(22) Anmeldetag: 21.04.2008
(51) Int. Cl.: A61K 31/137, A61P 25/04

(54) **Tapentadol zur Schmerzbehandlung bei Arthrose**

(30) Priorität: 23.04.2007 DE 102007019417
(62) Teilanmeldung aus: 08749015.7
(71) Anmelder: Grünenthal GmbH, 52078 Aachen (DE)
(72) Erfinder: Lange, Claudia, 40239 Düsseldorf (DE); Rombout, Ferdinand, 6343 CL Klimmen (NL)
(74) Vertreter: Kutzenberger Wolff & Partner

(57) **Zusammenfassung**

Die Erfindung betrifft die Verwendung von Tapentadol zur Behandlung von Schmerz bei Arthrose.

## Beschreibung

Die Erfindung betrifft die Verwendung von Tapentadol zur Behandlung von Schmerz bei Arthrose.

Arthrose (*Osteoarthritis, Arthrosis deformans*) ist die am weitesten verbreitete menschliche Gelenkerkrankung. Sie ist eine dynamische, aber langsam progrediente, degenerative Erkrankung des Knorpels und anderer Gelenkgewebe, vor allem bei älteren Individuen, mit intermittierenden entzündlichen Episoden. Aufgrund der fehlenden Entzündungsparameter, eingeschränkter Beweglichkeit, kurzfristiger Gelenksteifigkeit und radiologischer Merkmale kann sie von anderen rheumatischen Erkrankungen abgegrenzt werden.

Die Arthrose oder der Gelenkverschleiß ist ein Gelenkschaden, die mit einem Abbau des Gelenkknorpels beginnt. In schweren Fällen kommt es schließlich zu Umbauprozessen im benachbarten Knochen, die Gelenkoberfläche wird zerstört. Die Folgen der Erkrankung sind daher Schmerzen und Steifigkeit des Gelenks mit Bewegungseinschränkungen. Die Gelenke können sich verformen und schließlich ganz verknöchern. Eine Arthrose ist ein zumeist langsam fortschreitendes Geschehen. Die Knorpelschicht wird in Folge zuerst dicker und die Chondrozyten werden metabolisch aktiver. Veränderungen der subchondralen Trabekel führen zu einer verminderten Druckentlastung durch den spongiösen Knochen. Das Reparationsgewebe wird stärker belastet und mit fortschreitender Krankheitsdauer ändert sich das Gleichgewicht hinsichtlich einer Destruktion. Röntgenologisch wird eine Gelenkspaltverengung sichtbar und an den Rändern werden Osteophyten gebildet. Bezüglich weiterer Einzelheiten kann beispielsweise vollumfänglich verwiesen werden auf D. Höffler et al., AVP Therapieempfehlungen der Arzneimittelkommission der Deutschen Ärzteschaft, Arzneiverordnung in der Praxis, "Degenerative Gelenkerkrankungen", 2. Auflange 2001; und H. Bröll et al., CliniCum, Sonderausgabe September 2001, Konsensus-Statement, "Arthrose - Diagnostik % Therapie".

Prinzipiell können alle Gelenke von arthrotischen Veränderungen betroffen sein. Am meisten betroffen sind jedoch die Kniegelenke (Gonarthrose) und Hüftgelenke (Koxarthrose), auf denen viel Gewicht lastet. Häufig kommt die Erkrankung auch in den kleinen Wirbelsäulengelenken (Spondylarthrose) sowie in den Fingergelenken vor. Gemäß der ICD-10 sind Hüftund Knie-Arthrose als primäre Knorpelerkrankungen definiert, die mit schmerzhaften Bewegungseinschränkungen (Anlaufschmerz, Belastungsschmerz) bzw. Gehbehinderungen einhergehen. Eine Entzündung, wie eine Synovitis, kann, muss aber nicht etabliert sein.

Leit- und Frühsymptom von Arthrose sind Schmerzen (Frühtrias: Anlaufschmerz, Ermüdungsschmerz, Belastungsschmerz; Spättrias: Dauerschmerz, Nachtschmerz, Muskelschmerz). Sie werden von Bewegungseinschränkungen, Wetterfühligkeit, Krepitation begleitet. Schmerzursachen bei der Arthrose resultieren vorwiegend aus Reizzuständen in periartikulären Sehnen- und Bandansätzen, sekundären Entzündungen, Gelenkkapseldehnung, Reizergüssen, Druckerhöhung im subchondralen Knochen und Mikrofrakturen.

In frühen Stadien treten Schmerzen nur bei Belastung auf und lassen bei fortgeführter Bewegung, z.B. bei längerem Gehen, nach wenigen Minuten wieder nach. Tritt eine Entzündung hinzu, zeigen sich die typischen Beschwerden der aktivierten Arthrose: das Gelenk schmerzt, fühlt sich warm an und ist geschwollen. Die Beweglichkeit ist eingeschränkt. Oft klingt die Entzündung auch ohne Behandlung ab. Dies erklärt den meist schubweisen Verlauf der Arthrose: Phasen stärkerer Schmerzen und Bewegungseinschränkung wechseln sich mit Phasen geringer Schmerzhaftigkeit und guter Beweglichkeit ab. Je weiter die Abnutzungserscheinungen fortschreiten, desto rascher folgt eine Schmerzphase auf die andere. Schließlich bleibt der Schmerz andauernd bestehen.

In der Behandlung stehen mehrere nicht-medikamentöse und medikamentöse Alternativen zur Verfügung, die einzeln oder in Kombination zur Anwendung kommen:
- allgemeine Maßnahmen, z.B. Schwimmen, Radfahren, gezielte Gymnastik, Benutzung von Gehhilfen, Diät, etc.;
- physikalische Therapie, z.B. Wärmepackungen, Elektrotherapie und Bewegungstherapie, etc.;
- Pharmakotherapie;
- Orthopädie-Technik, z.B. Bandagen, Orthesen, etc.; und
- operative Therapie, z.B. Transplantation von autologen Knorpelzellen, künstlicher Gelenkersatz, etc..

Zur Beurteilung des Erfolges einer bestimmten Therapie empfiehlt die *European League Against Rheumatism* (EULAR) den *Lequesne Index,* d.h. die Gesamtbewertung durch den Arzt und die Schmerzeinschätzung des Patienten. Von der FDA werden neben der Bewertung von Schwellung, Rötung und Druckfestigkeit des Gelenks die Bewertung des Schmerzes und der Funktion mittels des *Western-Ontario-McMaster-Universities-Osteoarthritis-Index* (WOMAC)- und des *Lequesne-Indices* empfohlen. Die *Osteoarthritis Research Society* empfiehlt für Arzneimittel, die zur symptomatischen Behandlung der Arthrose eingesetzt werden, die Skalen des WOMAC-Schmerzscores als Hauptzielkriterium, als Nebenzielkriterien den Bewegungseinschränkungsscore des WOMAC oder den *Lequesne Index,* außerdem die Gesamtbeurteilung durch den Arzt und die Patienten.

Das pharmakotherapeutische Spektrum der zur Therapie der Arthrose angebotenen Wirkstoffgruppen umfasst
- nicht-opioide Analgetika, z.B. Paracetamol;
- nichtsteroidale Antirheumatika/Antiphlogistika (NSAR), z.B. Acemetacin, Acetylsalicylsäure, Aceclofenac, Diclofenac, Ibuprofen, Ketoprofen, Mefenaminsäure, Tiaprofensäure, Indometacin, Lonazolac, Naproxen, Proglumetacin, Meloxicam, Piroxicam, Rofecoxib, Celecoxib;
- Opioidanalgetika, z.B. Dihydrocodein, Tramadol, Tilidin-Naloxon, Morphin, Buprenorphin, Oxycodon, Fentanyl und Hydromorphon;
- perkutan applizierbare Antiphlogistika und Hyperämika;
- Glucocorticosteroid-Kristallsuspensionen für intraartikuläre Injektionen; und
- weitere Wirkstoffe zur oralen oder intraartikulären Injektionen, z.B. Glucosamin, Ademetionion, Oxaceprol, Hyaluronsäure, etc.

Opioidanalgetika gehören nicht zum Routine-Repertoire der medikamentösen Behandlung von Arthrose, sind aber in bestimmten Situationen unumgänglich. Herkömmliche Opioidanalgetika zeigen allerdings zum Teil erhebliche Nebenwirkungen, insbesondere Obstipation, Übelkeit, Erbrechen, Kopfschmerzen, Sedierung, Müdigkeit, Atemdepression, Allergien und mitunter Blutdruckabfall. Diese Nebenwirkungen erschweren eine Langzeittherapie chronischer Schmerzzustände bei Arthrose. Daher ergibt sich die Indikation für eine Behandlung mit herkömmlichen Opioidanalgetika meist erst nach Ausschöpfen aller anderen therapeutischen Möglichkeiten, beispielsweise bei Patienten, die nicht operationsfähig sind, aber an massiven, auf andere analgetisch wirkende Substanzen nicht ansprechende Ruheschmerzen leiden.

Es besteht ein Bedarf an alternativen pharmakotherapeutischen Behandlungsmethoden der Arthrose, welche sich durch eine wirksame Schmerzbekämpfung und ein vermindertes Nebenwirkungsprofil auszeichnen.

Aufgabe der Erfindung war es daher Verbindungen aufzufinden, welche bei der Schmerzbekämpfung bei Arthrose wirksam sind und Vorteile gegenüber herkömmlichen Analgetika zeigen.

Diese Aufgabe wird durch den Gegenstand der Patentansprüche gelöst.

Die Erfindung betrifft die Verwendung von Tapentadol zur Herstellung eines Medikamentes zur Behandlung von Schmerz bei Arthrose.

Es wurde überraschend gefunden, dass Tapentadol, vorzugsweise als *prolonged release* (PR) Formulierung (synonym zu *extended release* (ER) Formulierung), d.h. Formulierung mit anhaltender Freisetzung im Sinne des Europäischen Arzneibuchs, eine hervorragende Wirksamkeit bei der Behandlung von Schmerz bei Arthrose und ein verringertes Nebenwirkungsspektrum auf sich vereint. Unter anhanltender Freisetzung wird üblicherweise eine modifizierte Freisetzung verstanden, welche sich von der Freisetzung konventioneller Darreichungsformen unterscheidet, die über die gleiche Route verabreicht werden. Die Modifizierung der Freisetzung wird üblicherweise durch ein spezielles Design der Darreichungsform oder eine spezielle Herstellungsmethode erreicht.
Figur 1 zeigt eine schematische Darstellung des Titrationsschemas, welches bei der Untersuchung der Wirksamkeit von Tapentadol zur Behandlung von Schmerz bei Arthrose eingehalten wurde.
Figur 2 zeigt eine schematische Darstellung der Wirksamkeit von Tapentadol (100 mg und 200 mg) im Vergleich zu Placebo und Oxycodon.
Figur 3 zeigt eine mathematische Auswertung der Verteilung der Serumkonzentration innerhalb einer Patientenpopulation nach Verabreichung verschiedener Dosierungen von Tapentadol.
Figur 4 zeigt eine mathematische Auswertung des Zusammenhangs zwischen Serumkonzentration von Tapentadol und Wirkung im Hinblick auf die Schmerzlinderung in einer Patientenpopulation auf Grundlage von Daten aus verschiedenen klinischen Studien.

Tapentadol, d.h. (-)-(1 R,2R)-3-(3-Dimethylamino-1-ethyl-2-methyl-propyl)-phenol, ist ein synthetisches, zentral wirkendes Analgetikum, welches bei der Behandlung von mäßigem bis schwerem, akutem oder chronischem Schmerz wirksam ist.

Tapentadol zeigt einen zweifachen Wirkmechanismus, einerseits als µ-Opioid Rezeptor Agonist und andererseits als Noradrenalin Transporter Inhibitor. Beim Menschen ist die Affinität von Tapentadol zum rekombinant hergestellten µ-Opioid Rezeptor 18fach geringer als von Morphin. Klinische Studien haben jedoch gezeigt, dass die schmerzlindernde Wirkung von Tapentadol lediglich zwei- bis dreimal geringer als die von Morphin ist. Die nur geringfügig verminderte analgetische Wirksamkeit bei gleichzeitig 18fach verminderter Affinität zum rekombinanten µ-Opioid Rezeptor deutet darauf hin, dass die den Noradrenalin Transporter inhibierende Eigenschaft von Tapentadol ebenfalls zu seiner analgetischen Wirksamkeit beiträgt. Demzufolge ist davon auszugehen, dass Tapentadol eine ähnliche analgetische Wirksamkeit wie reine µ-Opioid Rezeptor Agonisten, jedoch weniger mit dem µ-Opioid Rezeptor im Zusammenhang stehende Nebenwirkungen zeigt. Die Verbindung kann in Form ihrer freien Base oder als Salz oder Solvat eingesetzt werden. Die Herstellung der freien Base ist beispielsweise aus EP-A 693 475 bekannt.

Zum Zwecke der Beschreibung bedeutet "Tapentadol" (-)-(1 R,2R)-3-(3-Dimethylamino-1-ethyl-2-methyl-propyl)-phenol, dessen pharmazeutisch verträglichen Salze und Solvate.

Geeignete pharmazeutisch verträgliche Salze umfassen Salze anorganischer Säuren, wie z.B. Chlorwasserstoff, Bromwasserstoff und Schwefelsäure, und Salze organischer Säuren, wie Methansulfonsäure, Fumarsäure, Maleinsäure, Essigsäure, Oxalsäure, Bernsteinsäure, Äpfelsäure, Weinsäure, Mandelsäure, Milchsäure, Zitronensäure, Glutaminsäure, Acetylsalicylsäure, Nikotinsäure, Aminobenzoesäure, α-Liponsäure, Hippursäure und Asparaginsäure. Das bevorzugte Salz ist das Hydrochlorid.

In einer bevorzugten Ausführungsform handelt es sich bei dem Medikament um eine feste Arzneiform. Vorzugsweise ist das Medikament zur oralen Verabreichung konfektioniert. Es sind jedoch auch andere Verabreichungsformen möglich, beispielsweise buccal, sublingual, transmucosal, rektal, intralumbal, intraperitoneal, transdermal, intravenös, intramuskulär, intragluteal, intrakutan und subkutan.

Je nach Konfektionierung enthält das Medikament vorzugsweise geeignete Zusatz- und/oder Hilfsstoffe. Geeignete Zusatz- und/oder Hilfsstoffe im Sinne der Erfindung sind alle dem Fachmann aus dem Stand der Technik bekannten Stoffe zur Erreichung galenischer Formulierungen. Die Auswahl dieser Hilfsstoffe sowie die einzusetzenden Mengen hängen davon ab, wie das Arzneimittel appliziert werden soll, d.h. oral, intravenös, intraperitoneal, intradermal, intramuskulär, intranasal, buccal oder lokal.

Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen.

Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc.

Für Suppositorien können u.a. Wachse bzw. Fettsäureester und für parenterale Applikationsmittel Trägerstoffe, Konservierungsmittel, Suspensionshilfsmittel etc. verwendet werden.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnussöl, Erdnussöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung dieser Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in *"*Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die Wirkstoff in homogener Verteilung enthält. Unter einer homogenen Verteilung wird hier verstanden, dass der Wirkstoff gleichmäßig über die gesamte Zusammensetzung verteilt ist, so dass diese ohne weiteres in gleich wirksame Einheitsdosis-Formen, wie Tabletten, Kapseln, Dragees unterteilt werden kann. Die feste Zusammensetzung wird anschließend in Einheitsdosis-Formen unterteilt. Die Tabletten oder Pillen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an Patienten zu verabreichenden Mengen an Tapentadol variieren in Abhängigkeit vom Gewicht des Patienten, von der Applikationsart und dem Schweregrad der Erkrankung. In einer bevorzugten Ausführungsform enthält das Medikament Tapentadol in einer Menge von 10 bis 300 mg, bevorzugter 20 bis 290 mg, noch bevorzugter 30 bis 280 mg, am bevorzugtesten 40 bis 260 mg, als Äquivalentdosis bezogen auf die freie Base.

Aus oral, rektal oder perkutan anwendbaren Zubereitungsformen kann Tapentadol verzögert freigesetzt werden. Bevorzugt ist das Medikament zur einmal täglichen Verabreichung, zur zweimal täglichen Verabreichung *(bid)* oder zur dreimal täglichen Verabreichung konfektioniert, wobei die zweimal tägliche Verabreichung *(bid)* besonders bevorzugt ist.

Eine verzögerte Freisetzung von Tapentadol kann beispielsweise durch Retardierung mit Hilfe einer Matrix, eines Überzugs oder osmotisch wirkender Freisetzungssysteme erreicht werden (vgl. z.B. US-A-2005-58706).

In einer bevorzugten Ausführungsform beträgt die mittlere Serumkonzentration von Tapentadol, nach zweimal täglicher Verabreichung des Medikaments über einen Zeitraum von wenigstens drei Tagen, bevorzugter wenigstens vier Tagen und insbesondere wenigstens fünf Tagen, durchschnittlich mindestens 5,0 ng/ml, mindestens 10 ng/ml, mindestens 15 ng/ml oder mindestens 20 ng/ml, bevorzugter mindestens 25 ng/ml oder mindestens 30 ng/ml, noch bevorzugter mindestens 35 ng/ml oder mindestens 40 ng/ml, am bevorzugtesten mindestens 45 ng/ml oder mindestens 50 ng/ml und insbesondere mindestens 55 ng/ml oder mindestens 60 ng/ml. Dies bedeutet, dass Tapentadol über einen Zeitraum von mindestens drei Tagen zweimal täglich verabreicht wird und dann, vorzugsweise 2 h nach der zuletzt erfolgten Verabreichung, die Serumkonzentration gemessen wird. Der maßgebliche Zahlenwert ergibt sich dann als Mittelwert aus der Gesamtheit der untersuchten Patienten.

In einer bevorzugten Ausführungsform beträgt die mittlere Serumkonzentration von Tapentadol bei höchstens 50% der Patientenpopulation, welche vorzugsweise wenigstens 100 Patienten umfasst, bevorzugter bei höchstens 40%, noch bevorzugter bei höchstens 30%, am bevorzugtesten bei höchstens 20% und insbesondere bei höchstens 10% der Patientenpopulation, nach zweimal täglicher Verabreichung über einen Zeitraum von wenigstens drei Tagen, bevorzugter wenigstens vier Tagen und insbesondere wenigstens fünf Tagen, durchschnittlich weniger als 5,0 ng/ml, bevorzugt weniger als 7,5 ng/ml, noch bevorzugter weniger als 10 ng/ml, am bevorzugtesten weniger als 15 ng/ml und insbesondere weniger als 20 ng/ml.

In einer bevorzugten Ausführungsform beträgt die mittlere Serumkonzentration von Tapentadol bei höchstens 50% der Patientenpopulation, welche vorzugsweise wenigstens 100 Patienten umfasst, bevorzugter bei höchstens 40%, noch bevorzugter bei höchstens 30%, am bevorzugtesten bei höchstens 20% und insbesondere bei höchstens 10% der Patientenpopulation, nach zweimal täglicher Verabreichung über einen Zeitraum von wenigstens drei Tagen, bevorzugter wenigstens vier Tagen und insbesondere wenigstens fünf Tagen, durchschnittlich mehr als 300 ng/ml, bevorzugter mehr als 275 ng/ml, noch bevorzugter mehr als 250 ng/ml, am bevorzugtesten mehr als 225 ng/ml und insbesondere mehr als 200 ng/ml.

Bevorzugt liegt die mittlere Serumkonzentration von Tapentadol bei wenigstens 50% oder 55% der Patientenpopulation, welche vorzugsweise wenigstens 100 Patienten umfasst, bevorzugter bei wenigstens 60% oder 65%, noch bevorzugter bei wenigstens 70% oder 75%, am bevorzugtesten bei wenigstens 80% oder 85% und insbesondere bei wenigstens 90% oder 95% der Patientenpopulation, nach zweimal täglicher Verabreichung über einen Zeitraum von wenigstens drei Tagen, bevorzugter wenigstens vier Tagen und insbesondere wenigstens fünf Tagen, durchschnittlich im Bereich von 1,0 ng/ml bis 500 ng/ml, bevorzugter im Bereich von 2,0 ng/ml bis 450 ng/ml, noch bevorzugter im Bereich von 3,0 ng/ml bis 400 ng/ml, am bevorzugtesten im Bereich von 4,0 ng/ml bis 350 ng/ml und insbesondere im Bereich von 5,0 ng/ml bis 300 ng/ml.

In einer bevorzugten Ausführungsform beträgt die prozentuale Standardabweichung (Variationskoeffizient) der mittleren Serumkonzentration von Tapentadol, vorzugsweise bei einer Patientenpopulation von 100 Patienten, nach zweimal täglicher Verabreichung des Medikaments über einen Zeitraum von wenigstens drei Tagen, bevorzugter wenigstens vier Tagen und insbesondere wenigstens fünf Tagen, höchstens ± 90%, bevorzugter höchstens ± 70%, noch bevorzugter höchstens ± 50%, höchstens ± 45% oder höchstens ± 40%, am bevorzugtesten höchstens ± 35%, höchstens ± 30% oder höchstens ± 25%und insbesondere höchstens ± 20%, höchstens ± 15% oder höchstens ± 10%.

Bevorzugt sind die Serumkonzentrationen Durchschnittswerte, welche sich aus Messungen bei einer Patientenpopulation von bevorzugt wenigstens 10, bevorzugter wenigstens 25, noch bevorzugter wenigstens 50, noch bevorzugter wenigstens 75, am bevorzugtesten wenigstens 100 und insbesondere wenigstens 250 Patienten ergeben. Einem Fachmann ist bekannt, wie die Serumkonzentrationen von Tapentadol bestimmt werden können. In diesem Zusammenhang kann beispielsweise verwiesen werden auf T.M. Tschentke et al., Drugs of the Future, 2006, 31 (12), 1053.

In einer bevorzugten Ausführungsform
- ist das Medikament zur oralen Verabreichung konfektioniert;
- ist das Medikament eine feste und/oder verpresste und/oder filmbeschichtete Arzneiform; und/oder
- setzt das Medikament Tapentadol aus einer Matrix verzögert frei; und/oder
- enthält das Medikament Tapentadol in einer Menge von 0,001 bis 99,999 Gew.-%, bevorzugter 0,1 bis 99,9 Gew.-%, noch bevorzugter 1,0 bis 99,0 Gew.-%, noch bevorzugter 2,5 bis 80 Gew.-%, am bevorzugtesten 5,0 bis 50 Gew.-% und insbesondere 7,5 bis 40 Gew.-%, bezogen auf das Gesamtgewicht des Medikaments; und/oder
- enthält das Medikament einen pharmazeutisch verträglichen Träger und/oder pharmazeutisch verträgliche Hilfsstoffe; und/oder
- weist das Medikament eine Gesamtmasse im Bereich von 25 bis 2.000 mg, bevorzugter 50 bis 1.800 mg, noch bevorzugter 60 bis 1.600 mg, noch bevorzugter 70 bis 1.400 mg, am bevorzugtesten 80 bis 1.200 mg und insbesondere 100 bis 1.000 mg auf, und/oder
- ist das Medikament ausgewählt aus der Gruppe bestehend aus Tabletten, Kapseln, Pellets und Granulaten.

Das Medikament kann als einfache Tablette und als überzogene Tablette (z.B. als Filmtablette oder Dragee) vorliegen. Die Tabletten sind üblicherweise rund und bikonvex, oblong Formen sind jedoch ebenfalls möglich. Granulate, Sphäroide, Pellets oder Mikrokapseln, welche in Sachets oder Kapseln gefüllt oder zu zerfallenden Tabletten verpresst sind, sind ebenfalls möglich.

Bevorzugt sind Medikamente, die wenigstens 0,001 bis 99,999 % Tapentadol enthalten, insbesondere niedrige, wirksame Dosierungen, um Nebenwirkungen zu vermeiden. Das Medikament enthält bevorzugt 0,01 Gew.-% bis 99,99 Gew.-% Tapentadol, bevorzugter 0,1 bis 90 Gew.-%, noch bevorzugter 0,5 bis 80 Gew.-%, am bevorzugtesten 1,0 bis 50 Gew.-% und insbesondere 5,0 bis 20 Gew.-%. Zur Vermeidung von Nebenwirkungen kann es von Vorteil sein, zu Beginn der Behandlung die zu verabreichende Menge an Tapentadol schrittweise zu erhöhen (Titration), um den Organismus langsam an den Wirkstoff zu gewöhnen. Vorzugsweise wird Tapentadol zunächst in einer Dosis verabreicht, welche unterhalb der analgetisch wirksamen Dosis liegt.

Besonders bevorzugt handelt es sich bei dem Medikament um eine orale Darreichungsform, welche zur zweimal täglichen Verabreichung konfektioniert ist und Tapentadol in einer Menge von 20 bis 260 mg enthält, als Äquivalentdosis bezogen auf die freie Base.

In einer bevorzugten Ausführungsform handelt es sich bei dem Medikament um eine orale Darreichungsform mit sofortiger Freisetzung von Tapentadol (*immediate release*).

Erfindungsgemäß wird Tapentadol zur Behandlung von Schmerz bei Arthrose verwendet. Bevorzugt ist die Arthrose ausgewählt aus der Gruppe bestehend aus Gonarthrose, Koxarthrose und Spondylarthrose.

Bevorzugt handelt es sich bei der schmerzenden Arthrose um Arthrose im Sinne der ICD-10 (Internationale statistische Klassifikation der Krankheiten und verwandter Gesundheitsprobleme, WHO Ausgabe, vorzugsweise Stand 2007). Bevorzugt ist die Arthrose ausgewählt aus Polyarthrose [M15], Koxarthrose [M16], Gonarthrose [M17], Rhizarthrose [M18], Sonstige Arthrose [M19] und Arthrose der Wirbelsäule [M47]. Die in Klammern angegebenen Bezeichnungen beziehen sich auf die in der ICD-10 verwendete Nomenklatur.

Handelt es sich bei der Arthrose um Polyarthrose [M15], so ist diese vorzugsweise ausgewählt aus der Gruppe bestehend aus primärer, generalisierter (Osteo-)Arthrose [M15.0], Heberden-Knoten (mit Arthropathie) [M15.1], Bouchard-Knoten (mit Arthropathie) [M15.2], sekundärer, multipler Arthrose (posttraumatische Polyarthrose) [M 15.3], erosiver (Osteo-)Arthrose [M15.4], sonstiger Polyarthrose [M15.8] und nicht näher bezeichneter Polyarthrose (generalisierter (Osteo-)Arthrose ohne nähere Angabe) [M15.9].

Handelt es sich bei der Arthrose um Koxarthrose [M16], so ist diese vorzugsweise ausgewählt aus der Gruppe bestehend aus beidseitiger primärer Koxarthrose [M16.0], sonstiger primärer Koxarthrose (einseitig oder ohne nähere Angabe) [M16.1], beidseitiger Koxarthrose als Folge einer Dysplasie [M16.2], sonstige dysplasische Koxarthrose (einseitig oder ohne nähere Angabe) [M16.3], beidseitiger, posttraumatischer Koxarthrose [M16.4], sonstiger posttraumatischer Koxarthrose [M16.5] (einseitig oder ohne nähere Angabe), sonstiger, beidseitiger sekundärer Koxarthrose [M16.6], sonstiger sekundärer Koxarthrose (einseitig oder ohne nähere Angabe) [M16.7] und nicht näher bezeichneter Koxarthrose [M16.9].

Handelt es sich bei der Arthrose um Gonarthrose [M17], so ist diese vorzugsweise ausgewählt aus der Gruppe bestehend aus beidseitiger primärer Gonarthrose [M17.0], sonstiger primärer Gonarthrose (einseitig oder ohne nähere Angabe) [M17.1], beidseitiger, posttraumatischer Gonarthrose [M17.2], sonstiger posttraumatischer Gonarthrose [M17.3] (einseitig oder ohne nähere Angabe), sonstiger, beidseitiger sekundärer Gonarthrose [M17.4], sonstiger sekundärer Gonarthrose (einseitig oder ohne nähere Angabe) [M17.5] und nicht näher bezeichneter Gonarthrose [M17.9].

Handelt es sich bei der Arthrose um Rhizarthrose [M18], so ist diese vorzugsweise ausgewählt aus der Gruppe bestehend aus beidseitiger primärer Rhizarthrose [M18.0], sonstiger primärer Rhizarthrose (einseitig oder ohne nähere Angabe) [M18.1], beidseitiger, posttraumatischer Rhizarthrose [M18.2], sonstiger posttraumatischer Rhizarthrose [M18.3] (einseitig oder ohne nähere Angabe), sonstiger, beidseitiger sekundärer Rhizarthrose [M18.4], sonstiger sekundärer Rhizarthrose (einseitig oder ohne nähere Angabe) [M18.5] und nicht näher bezeichneter Rhizarthrose [M18.9].

Handelt es sich bei der Arthrose um Sonstige Arthrose [M19], so ist diese vorzugsweise ausgewählt aus der Gruppe bestehend aus primärer Arthrose sonstiger Gelenke (primäre Arthrose ohne nähere Angabe) [M19.0], posttraumatischer Arthrose sonstiger Gelenke (posttraumatische Arthrose ohne nähere Angabe) [M19.1], sonstiger sekundärer Arthrose (sekundäre Arthrose ohne nähere Angabe) [M19.2], sonstiger näher bezeichneter Arthrose [M19.8] und nicht näher bezeichneter Arthrose [M19.9].

Bevorzugt ist der Schmerz mäßig bis stark. In einer bevorzugten Ausführungsform ist der Schmerz ausgewählt aus der Gruppe bestehend aus Anlaufschmerz, Belastungsschmerz, Ermüdungsschmerz, periartikulärem Druckschmerz, ausstrahlendem Schmerz (z.B. Knieschmerz bei bestehender Koxarthrose), Ruheschmerz nach längerem Verharren in gleicher Stellung, Dauerschmerz, Spontanschmerz, Bewegungsschmerz, Nachtschmerz, Muskelschmerz, Endlagenschmerz, ossärem Schmerz als Spontan- und Ruheschmerz.

Auch wenn die erfindungsgemäßen Medikamente lediglich geringe Nebenwirkungen zeigen, kann es beispielsweise zur Vermeidung von bestimmten Formen der Abhängigkeit von Vorteil sein, neben Tapentadol auch Morphinantagonisten, insbesondere Naloxon, Naltrexon und/oder Levallorphan, zu verwenden.

Ferner betrifft die Erfindung ein Verfahren zur Behandlung von Schmerz bei Arthrose, bei dem Tapentadol in einer pharmazeutisch verträglichen Menge an einen Patienten verabreicht wird.

Die folgenden Beispiele dienen zur näheren Erläuterung der Erfindung, sind jedoch nicht einschränkend auszulegen.

### Beispiel 1:

### Ziel:

Die Wirksamkeit und die Verträglichkeit von Tapentadol mit anhaltender Freisetzung (*prolonged release* (PR)) und Oxycodon HCl mit kontrollierter Freisetzung (*controlled release* (CR)) wurden mit Placebo verglichen bei Patienten mit mittlerem bis schwerem Schmerz bei Arthrose des Knies.

### Methoden (randomisierte, Placebo kontrollierte Doppelblindstudie):

Patienten (N = 670) wurden zufällig ausgewählt und über 28 Tage zweimal täglich entweder mit Tapentadol PR 100 mg, mit Tapentadol PR 200 mg, mit Oxycodon HCl CR 20 mg bzw. mit Placebo behandelt. Zu Beginn der Behandlung wurde die Dosis titriert. Der primäre Wirksamkeitsendpunkt war das durchschnittliche Schmerzempfinden während der vorangegangenen 24 Stunden zum Zeitpunkt der letzten ärztlichen Untersuchung (*final visit*) auf Basis einer visuellen, analogen 100-mm Skala (VAS, 0 mm = kein Schmerz, 100 mm = stärkster, vorstellbarer Schmerz).

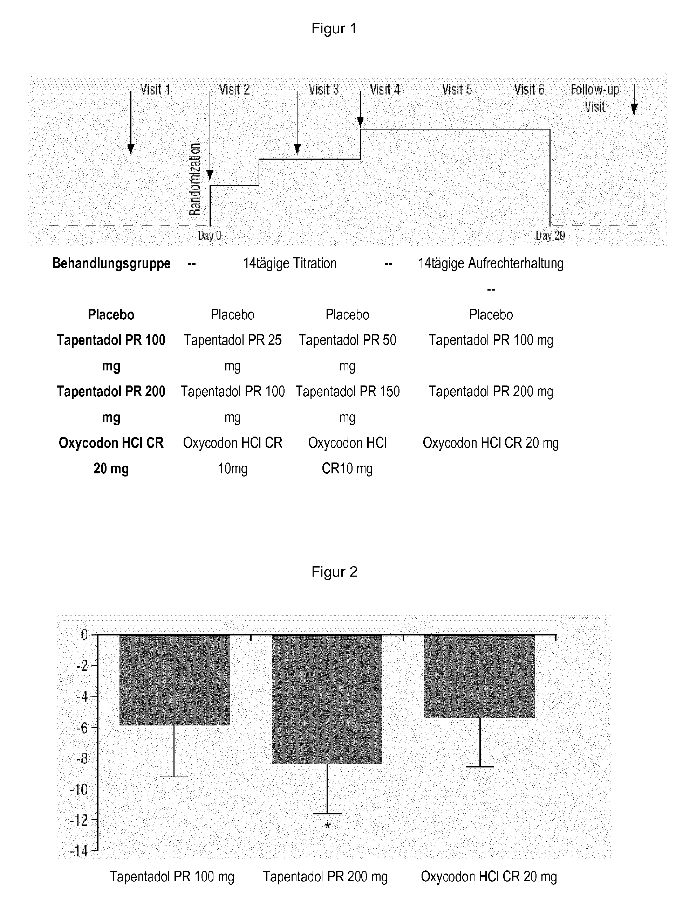

Die Studie bestand aus einer 14tägigen, Doppelblind-Titrationsphase (3 Tage -> 11 Tage), an die sich eine 14tägige Doppelblind-Aufrechterhaltungsphase anschloss (bei der jeweils höchsten Dosierung des Titrationsschemas; vgl. Figur 1):
- Tapentadol PR 100 mg: 25 mg (*bid*) *->* 50 mg (*bid*) *->* 100 mg (*bid*);
- Tapentadol PR 200 mg: 100 mg (*bid*) *->* 150 mg (*bid*) -> 200 mg (*bid*);
- Oxycodon HCl CR 20 mg: 10 mg (*bid*) -> 10 mg (*bid*) -> 20 mg (*bid*).

### Ergebnisse:

Der Unterschied der angepassten, mittleren Fehlerquadrate (± Standard Fehler) in der durchschnittlichen Schmerzintensität gegenüber Placebo war für Tapentadol PR 200 mg signifikant (-8.4 mm [±3.30]; P = 0.021). Die Unterschiede der angepassten mittleren Fehlerquadrate (± Standard Fehler) in der durchschnittlichen Schmerzintensität gegenüber Placebo war für Tapentadol PR 100 mg -5.9 mm (±3.34; P = 0.142) und für Oxycodon HCl CR 20 mg -5.4 mm (±3.22; P = 0.091), d.h. Tapentadol PR 100 und Oxycodon HCl CR 20 mg zeigten ein ähnliches Verhalten (vgl. Figur 2).

In allen Gruppen waren gastrointestinale Beschwerden (einschließlich Übelkeit, Verstopfung und Erbrechen) und Beschwerden des Nervensystems (einschließlich Müdigkeit und Schwindelgefühl) die am häufigsten auftretenden Nebenwirkungen:

| Nebenwirkungen | Placebo | Tapentadol PR 100 mg | Tapentadol PR 200 mg | Oxycodon HCl CR 20 mg |
|---|---|---|---|---|
| Gastrointestinal | 23% | 30% | 49% | 56% |
| Verstopfung | 5% | 7% | 10% | 20% |
| Nervensystem | 15% | 24% | 34% | 43% |

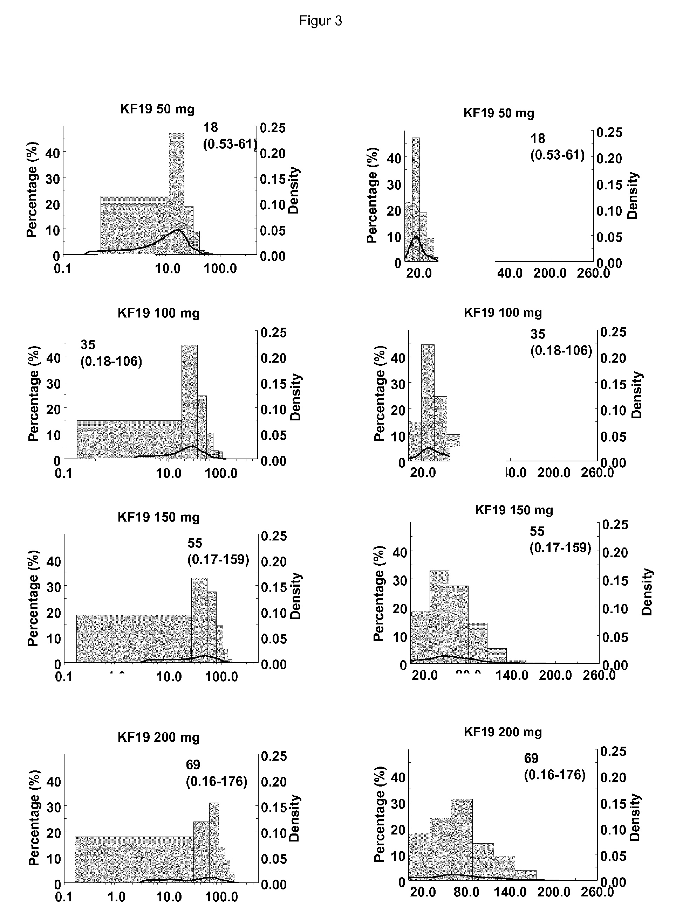

Eine mathematische Auswertung der Verteilung der Serumkonzentration innerhalb einer Patientenpopulation nach Verabreichung verschiedener Dosierungen von Tapentadol ist in Figur 3 dargestellt.

Die klinischen Daten belegen, dass Tapentadol PR 200 mg für 4 Wochen wirksam bei der Behandlung von mittlerem bis schwerem chronischem Schmerz bei Arthrose ist. Im Hinblick auf gastrointestinale Nebenwirkungen und Nebenwirkungen, welche mit dem zentralen Nervensystem zusammenhängen, deuten die klinischen Daten auf eine verbesserte Verträglichkeit von Tapentadol im Vergleich zu Oxycodon HCl hin.

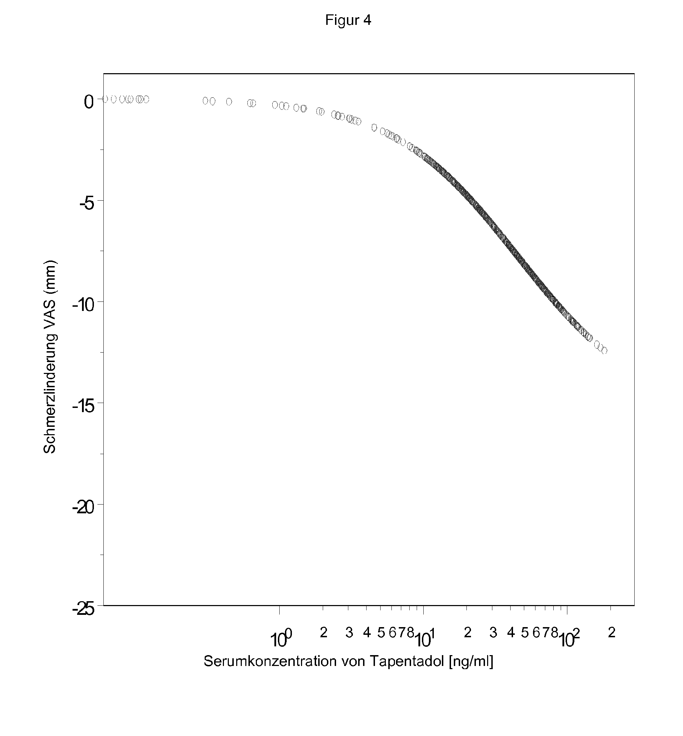

Eine mathematische Auswertung des Zusammenhangs zwischen Serumkonzentration von Tapentadol und Wirkung im Hinblick auf die Schmerzlinderung in einer Patientenpopulation auf Grundlage von Daten aus verschiedenen klinischen Studien ist in Figur 4 dargestellt.

### Beispiele 2 bis 4:

### Ziel:

Die Wirksamkeit und die Verträglichkeit von Tapentadol mit sofortiger Freisetzung (*immediate release* (IR)) und Oxycodon HCl mit sofortiger Freisetzung (*immediate release* (IR)) wurden mit Placebo verglichen bei Patienten mit mittlerem bis schwerem Schmerz bei Arthrose des Knies oder der Hüfte.

### Beispiel 2:

### Methoden (randomisierte, Doppelblindstudie, 90-Tage-Phase III, aktivkontrolliert, flexible Dosierung)

Patienten (N=878) erhielten zufällig in einem Verhältnis von 4:1 Tapentadol IR (50 oder 100 mg alle 4 bis 6 Stunden wie erforderlich; bis zu 600 mg/Tag) oder Oxycodon HCl IR (10 oder 15 mg alle 4 bis 6 Stunden wie erforderlich; bis zu 90 mg/Tag).

Die Schmerzintensität während der 24 Stunden vor jedem Besuch wurden vom ersten Tag der Medikation bis zum letzten Tag gemäß einer 11-fach unterteilten Bewertungsskala aufgezeichnet (0=kein Schmerz, 10=stärkster möglicher Schmerz). Die Verträglichkeit wurde vom ersten Tag der Medikation bis zum zweiten Tag nach der letzten Medikation der Studie bewertet.

### Ergebnisse:

Insgesamt wurden 679 Patienten in der Tapentadol IR Gruppe und 170 Patenten in der Oxycodon HCl IR Gruppe in die Analyse der Wirksamkeit und Sicherheit einbezogen.

Die Schmerzintensitäten waren über die Zeitdauer zwischen beiden Gruppen ähnlich. Die mittlere Grundlinienschmerzintensität betrug 7,0 für die Tapentadol IR Gruppe und 7,2 für die Oxycodon HCl IR Gruppe. Diese Werte nahmen gegen Ende der Doppelblindperiode auf 4,9 und 5,2 für die Tapentadol IR Gruppe bzw. für die Oxycodon HCl IR Gruppe ab. Die am häufigsten auftretenden Nebenwirkungen waren Übelkeit, Erbrechen, Schwindelgefühl, Verstopfung, Kopfschmerz und Müdigkeit. Die Patienten in der Tapentadol IR Gruppe zeigten ein signifikant (P < 0,001 für alle Messungen) geringeres Auftreten von Übelkeit (18%), Erbrechen (17 %) und Verstopfung (13 %) im Vergleich zu der Oxycodon HCl IR Gruppe (Übelkeit 29 %; Erbrechen 30 %; Verstopfung 27 %), wohingegen das Auftreten von Müdigkeit, Schwindelgefühl und Kopfschmerzen in beiden Gruppen ähnlich war.

Schwere Nebenwirkungen wurden bei 0,7 % der Patienten in der Tapentadol IR Gruppe und bei 1,8 % der Patienten in der Oxycodon HCl IR Gruppe berichtet. Diese wurden jedoch nicht auf den eingesetzten Wirkstoff zurückgeführt.

### Beispiel 3:

### Methoden (randomisierte Doppelblindstudie, Phase III)

878 Patienten wurden zufällig Tapantadol IR (50 oder 100 mg; maximal 600 mg/Tag) oder Oxycodon HCl IR (aktive Kontrolle; 10 oder 15 mg; maximal 90 mg/Tag) alle 4 bis 6 Stunden wie erforderlich über 90 Tage verabreicht. Die Behandlungsgruppen wurden mit Hilfe des Cochran-Mantel-Haenszel-Tests verglichen.

### Ergebnisse:

Die Analyse umfasste 679 Patienten in der Tapentadol IR Gruppe und 170 Patienten in der Oxycodon HCl IR Gruppe. Patienten mit Opioiderfahrung (d. h. Patienten, welche während 30 Tagen vor der Untersuchung zumindest 5 Tage die Woche ein Opioid zu sich genommen hatten) machten in der Tapentadol IR Gruppe 49,0 % und in der Oxycodon HCl IR Gruppe 48,2 % aus. Die mittlere Punktzahl für die Schmerzen nahm von der Basislinie bis zum Ende der Studie für Tapentadol IR von 7,0 auf 4,9 und für Oxycodon HCl IR von 4,2 auf 5,2 ab. Die am häufigsten auftretenden Nebenwirkungen waren Übelkeit, Erbrechen, Schwindelgefühl, Verstopfung, Kopfschmerzen und Müdigkeit. In der Tapentadol IR Gruppe traten signifikant (P < 0,001) weniger gastrointestinale Nebenwirkungen auf (Übelkeit, 18 %; Erbrechen, 17 %; Verstopfung, 13%) als in der Oxycodon HCl IR Gruppe (Übelkeit, 29 %; Erbrechen, 30 %; Verstopfung, 27 %), wohingegen das Auftreten von Kopfschmerzen, Schwindelgefühl und Müdigkeit in beiden Gruppen vergleichbar war. Im Allgemeinen hatten Patienten, welche nicht an Opioide gewöhnt waren, mehr Nebenwirkungen, diese Tendenz war jedoch für Tapentadol IR geringer ausgeprägt als für Oxycodon HCl IR.

Bei Patienten ohne Opioiderfahrung trat Erbrechen in der Tapentadol IR Gruppe in 18 % der Fälle und in der Oxycodon HCl IR in 39 % der Fälle auf, wohingegen Übelkeit in der Tapentadol IR Gruppe in 22 % der Fälle und in der Oxycodon HCl IR Gruppe in 35 % der Fälle berichtet wurde. Bei Patienten mit Opioiderfahrung wurde Erbrechen in der Tapentadol IR Gruppe in 16 % der Fälle und in der Oxycodon HCl IR Gruppe in 21 % der Fälle berichtet, wohingegen Übelkeit in der Tapentadol IR Gruppe in 14 % der Fälle und in der Oxycodon HCl IR in 23 % der Fälle auftrat. Opioiderfahrung führte in keiner der beiden Gruppen zu einer Verringerung des Auftretens von Verstopfung (Tapentadol IR: Opioiderfahrung, 12 %; keine Opioiderfahrung, 14%)(Oxycodon HCl IR: Opioiderfahrung,
27 %; keine Opioiderfahrung, 27%)

### Beispiel 4:

### Methoden (randomisierte, placebokontrollierte Doppelblindstudie, aktivkontrolliert, Phase III)

674 Patienten wurde zufällig Placebo, Tapentadol IR 50 oder 75 mg, Oxycodon HCl IR 10 mg alle 4 bis 6 Stunden während der Aufwachstunden verabreicht. Die Endpunkte der Studie umfassten die Summe der Schmerzintensitäten (SPID) über 5 Tage (primärer Endpunkt), Bewertung der Verträglichkeit und Analyse des Alters und Geschlechts, um potentielle Unterschiede zwischen den Untergruppen der Population zu untersuchen.

### Ergebnisse:

666 zufällig zugeordnete Patienten wurden in die Sicherheitsanalyse einbezogen; 659 Patienten wurden in die Wirksamkeitsanalyse einbezogen. Tapentadol IR 50 und 75 mg zeigte im Vergleich zu Placebo eine signifikante Verbesserung in der Schmerzlinderung auf Grundlage einer 5-Tage SPID Punktebewertung (P < 0,001). Die Oxycodon HCl IR 10 mg Gruppe zeigte ebenfalls signifikante Verbesserungen hinsichtlich der 5-Tage SPID Punktebewertung (P < 0,001) im Vergleich zur Placebogruppe, was die Empfindlichkeit des Assays bestätigt. Auf Grundlage von zuvor spezifizierten Kriterien für die 5-Tage SPID war Tapentadol IR 50 und 75 mg zumindest so wirksam wir Oxycodon HCl IR 10 mg. 5-Tage SPID Punktewerte waren in allen Gruppen der aktiven Behandlung ähnlich zwischen Patienten < 65 und ≥ 65 Jahren, sowie zwischen den männlichen und weiblichen Untergruppen. Gemeinsame Nebenwirkungen umfassten gastrointestinale Nebenwirkungen und zentralnervöse Nebenwirkungen. Insgesamt zeigte das Auftreten von gastrointestinalen Nebenwirkungen für Tapentadol IR 50 und 75 mg eine Dosisabhängigkeit (29 % bzw. 40 %), welche geringer als bei Oxycodon HCl IR 10 mg war (69 %). Dieser Trend konnte auch innerhalb der Untergruppen beobachtet werden. Patienten < 65 und ≥ 65 berichteten bei Tapentadol IR 50 mg über weniger gastrointestinale Nebenwirkungen (25 % bzw. 36 %) als bei 75 mg (42 % bzw. 38 %), und beide waren geringer als bei Oxycodon HCl IR 10 mg (66 % bzw. 74 %). Bei den männlichen und weiblichen Untergruppen wurden gastrointestinale Nebenwirkungen bei Tapentadol IR 50 mg in 21 % bzw. 39 % der Fälle berichtet, bei Tapentadol IR 75 mg in 28 % bzw. 54 % der Fälle, im Vergleich zu 58 % bzw. 81 % für Oxycodon HCl IR 10 mg.

### Schlussfolgerungen:

Die klinischen Daten belegen, dass Tapentadol IR wirksam bei der Behandlung von mittlerem bis schwerem chronischem Schmerz bei Arthrose ist. Im Hinblick auf gastrointestinale Nebenwirkungen deuten die klinischen Daten auf eine verbesserte Verträglichkeit von Tapentadol im Vergleich zu Oxycodon HCl hin.

## Patentansprüche

1. Verwendung von Tapentadol zur Herstellung eines Medikamentes zur Behandlung von Schmerz bei Arthrose.

2. Verwendung nach Anspruch 1, **dadurch gekennzeichnet, dass** die mittlere Serumkonzentration von Tapentadol, nach zweimal täglicher Verabreichung über einen Zeitraum von wenigstens drei Tagen, durchschnittlich mindestens 5,0 ng/ml beträgt.

3. Verwendung nach Anspruch 1 oder 2, **dadurch gekennzeichnet, dass** die mittlere Serumkonzentration von Tapentadol bei höchstens 50% der Patientenpopulation, nach zweimal täglicher Verabreichung über einen Zeitraum von wenigstens drei Tagen, durchschnittlich weniger als 5,0 ng/ml beträgt.

4. Verwendung nach einem der vorstehenden Ansprüche, **dadurch gekennzeichnet, dass** die mittlere Serumkonzentration von Tapentadol bei höchstens 50% der Patientenpopulation, nach zweimal täglicher Verabreichung über einen Zeitraum von wenigstens drei Tagen, durchschnittlich mehr als 300 ng/ml beträgt.

5. Verwendung nach einem der vorstehenden Ansprüche, **dadurch gekennzeichnet, dass** die mittlere Serumkonzentration von Tapentadol bei wenigstens 50% der Patientenpopulation, nach zweimal täglicher Verabreichung über einen Zeitraum von wenigstens drei Tagen, durchschnittlich im Bereich von 1,0 ng/ml bis 500 ng/ml liegt.

6. Verwendung nach einem der vorstehenden Ansprüche, **dadurch gekennzeichnet, dass** das Medikament eine feste Arzneiform ist.

7. Verwendung nach einem der vorstehenden Ansprüche, **dadurch gekennzeichnet, dass** das Medikament zur oralen Verabreichung konfektioniert ist.

8. Verwendung nach einem der vorstehenden Ansprüche, **dadurch gekennzeichnet, dass** das Medikament zur zweimal täglichen Verabreichung *(bid)* konfektioniert ist.

9. Verwendung nach einem der vorstehenden Ansprüche, **dadurch gekennzeichnet, dass** das Medikament Tapentadol in einer Menge von 10 bis 300 mg enthält.

10. Verwendung nach einem der vorstehenden Ansprüchen, **dadurch gekennzeichnet, dass** das Medikament
- einen pharmazeutisch verträglichen Träger enthält; und/oder
- eine Gesamtmasse im Bereich von 25 bis 2.000 mg aufweist; und/oder
- ausgewählt ist aus der Gruppe bestehend aus Tabletten, Kapseln, Pellets und Granulaten.

11. Verwendung nach einem der vorstehenden Ansprüche, **dadurch gekennzeichnet, dass** die Arthrose ausgewählt ist aus der Gruppe bestehend aus Gonarthrose, Koxarthrose und Spondylarthrose.

12. Verwendung nach einem der vorstehenden Ansprüche, **dadurch gekennzeichnet, dass** der Schmerz mäßig bis stark ist.

13. Verwendung nach einem der vorstehenden Ansprüche, **dadurch gekennzeichnet, dass** der Schmerz ausgewählt ist aus der Gruppe bestehend aus Anlaufschmerz, Belastungsschmerz, Ermüdungsschmerz, periartikulärem Druckschmerz, ausstrahlendem Schmerz, Ruheschmerz nach längerem Verharren in gleicher Stellung, Dauerschmerz, Spontanschmerz, Bewegungsschmerz, Nachtschmerz, Muskelschmerz, Endlagenschmerz und ossärem Schmerz als Spontan- und Ruheschmerz.
